# EUROPÄISCHE PATENTSCHRIFT

(11) **EP 0 361 119 B1**
(45) Veröffentlichungstag und Bekanntmachung des Hinweises auf die Patenterteilung: **27.10.1993**
(21) Anmeldenummer: 89116071.5
(22) Anmeldetag: 31.08.1989
(51) Int. Cl.: C07C 49/83, C07C 45/45

(54) **Verfahren zur Herstellung von Benzophenonen**
Process for the production of benzophenones
Procédé de préparation de benzophénones

(30) Priorität: 13.09.1988 DE 3831092
(43) Veröffentlichungstag der Anmeldung: 04.04.1990
(73) Patentinhaber: BASF Aktiengesellschaft, 67063 Ludwigshafen (DE)
(72) Erfinder: Neumann, Peter, D-6800 Mannheim 31 (DE); Eichenauer, Ulrich, Dr., D-6000 Frankfurt 70 (DE)

(56) Entgegenhaltungen:
- EP-A- 0 057 503
- EP-A- 0 069 598
- DE-A- 1 643 433
- FR-A- 1 375 033
- CHEMISCHE BERICHTE, 87. Jahrgang, 1954, Verlag Chemie, Weinheim/Bergstrasse, K. KINDLER et al: "Studien über den Mechanismus chemischer Reaktionen, XV. Mitteil.: "Über die Synthese von hologenierten Acylphenolen mittels freier Carbonsäuren", S. 194-202

## Beschreibung

Die vorliegende Erfindung betrifft ein neues Verfahren zur Herstellung von Benzophenonen durch Umsetzung von Benzoesäuren und Aromaten aus der Benzolreihe in Gegenwart von Bortrifluorid, wobei man die Umsetzung in Nitrobenzol als Reaktionsmedium durchführt.

Die Verwendung von Bortrifluorid als aktivierendes Reagenz für die Kondensation von Carbonsäuren mit reaktiven Aromaten zu aromatischen Ketonen ist bekannt und beispielsweise in Chem. Ber. 66, 411 (1933) ; Chem. Ber. 87, 194 (1954); Archiv Pharm. 287, 210 (1954) oder Pharmazie 1954, 102, beschrieben. Üblicherweise wird dabei die Carbonsäure, gelegentlich aber auch deren Reaktionspartner, als Lösungsmittel verwendet. Dies ist bei wertvollen Reaktanden unwirtschaftlich und bedeutet außerdem bei Reaktionspartnern, die in festem Aggregatzustand vorliegen, daß die Reaktion in einer Schmelze durchgeführt werden muß, was einen erheblichen technischen Aufwand mit sich bringt.

Um diese Nachteile zu vermeiden, wurde die Anwendung von Verdünnungsmitteln vorgeschlagen. So lehrt z.B. die FR-A-1 375 033 die Herstellung von Benzophenonen durch Umsetzung von Bortrifluorid-Komplexen der Reaktanden in Trichlorethylen als Verdünnungsmittel. Dabei müssen die jeweiligen Bortrifluorid-Komplexe aber in einer separaten Stufe hergestellt werden. Außerdem gilt Trichlorethylen als toxikologisch bedenklich.

Die JP-A-190 943/1984 beschreibt die Verwendung von Chlorbenzol als Reaktionsmedium. Hierbei ist jedoch die geringe Löslichkeit der Bortrifluorid-Komplexe der Reaktionspartner in diesem Medium von Nachteil. Sie führt nämlich zu verlängerten Reaktionszeiten und schlechteren Ausbeuten.

In der EP-A-69 598 wird unter anderem die Herstellung eines Dihydroxybenzophenons durch Umsetzung von 4-Hydroxybenzoesäure mit Phenol in wasserfreiem Fluorwasserstoff beschrieben.

Aufgabe der vorliegenden Erfindung war es nun, ein neues Verfahren zur Herstellung von Benzophenonen bereitzustellen, bei dem ebenfalls Bortrifluorid als Katalysator verwendet wird, bei dem aber die obengenannten Nachteile vermieden werden.

Es wurde nun gefunden, daß die Herstellung von Benzophenonen der Formel I 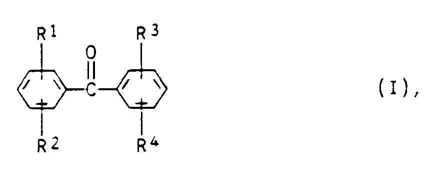
in der
- R¹ und R²: gleich oder verschieden sind und unabhängig voneinander jeweils Wasserstoff oder Hydroxy und
- R³ und R⁴: jeweils Hydroxy bedeuten,
durch Umsetzung von Benzoesäuren der Formel II 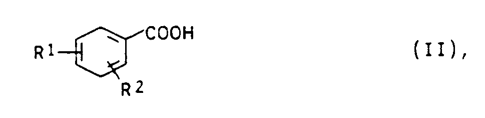
in der R¹ und R² jeweils die obengenannte Bedeutung besitzen, mit Aromaten aus der Benzolreihe, die der Formel III 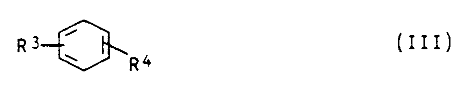
entsprechen, worin R³ und R⁴ jeweils die obengenannte Bedeutung besitzen, in Gegenwart von Bortrifluorid als Katalysator vorteilhaft gelingt, wenn man die Umsetzung in Nitrobenzol als Reaktionsmedium durchführt.

Geeignete Aromaten der Formel III sind beispielsweise Resorcin oder Hydrochinon.

Das erfindungsgemäße Verfahren wird zweckmäßig so durchgeführt, daß man die Benzoesäure der Formel II und den Aromaten aus der Benzolreihe (Formel III) in Nitrobenzol vorlegt und in dieses Gemisch üblicherweise bei Raumtemperatur, Bortrifluorid unter Rühren eingast. Dann folgt die Kondensationsreaktion, die unter Rühren bei einer Temperatur von 20 bis 200°C, vorzugsweise 20 bis 130°C und insbesondere 50 bis 90°C vorgenommen wird. Die Reaktion ist im allgemeinen 0,5 bis 5 Stunden beendet.

Zur Aufarbeitung kann man das Reaktionsgemisch z.B. einer Wasserdampfdestillation unterwerfen, um das Nitrobenzol zu entfernen. Nach dem Abkühlen kristallisiert dabei das Benzophenon der Formel I in der Regel aus der verbleibenden wäßrigen Mutterlauge aus. Das mittels Wasserdampf entfernte Nitrobenzol kann nach Regenerierung wieder in die Reaktion zurückgeführt werden.

Eine bevorzugte Aufarbeitungsmethode besteht darin, das Reaktionsgemisch in einem Flüssig-Flüssig-Extraktor bei erhöhter Temperatur mit Wasser zu extrahieren, wobei die Extraktion drucklos oder unter Druck durchgeführt werden kann. Die Extraktionstemperatur liegt im allgemeinen zwischen 80°C und dem Siedepunkt des Wassers beim angewandten Druck, vorzugsweise bei 90 bis 100°C. Der Druck, bei dem die Extraktion durchgeführt wird, kann zwischen 0,9 und 10 bar eingestellt werden, bevorzugt ist Normaldruck. Die Menge des zur Extraktion verwandten Wassers kann zwischen 50 und 1000 Gew.-%, bezogen auf das verwendete Nitrobenzol, gewählt werden. Die Extraktion kann diskontinuierlich, bevorzugt aber kontinuierlich durchgeführt werden. Besonders bevorzugt ist eine kontinuierliche Arbeitsweise, bei der Wasser aus dem wäßrigen Extrakt verdampft und anschließend erneut zur Extraktion verwandt wird.

Zur Isolierung wird der wäßrige Extrakt abgekühlt und das Zielprodukt durch Filtration isoliert. Gegebenenfalls kann auch aus der abgekühlten organischen Phase Wertprodukt durch Filtrieren isoliert werden. Die Filtration der wäßrigen Phase kann, unabhängig von der Extraktion, kontinuierlich oder diskontinuierlich durchgeführt werden.

Verglichen mit der Wasserdampfdestillation, ergibt die extraktive Aufarbeitung noch bessere Ausbeuten und eine noch bessere farbliche Qualität des Zielprodukts. Ein weiterer Vorteil besteht darin, daß weniger Wasser mit Nitrobenzol kontaminiert wird. Das ist von ökologischem Interesse.

Im erfindungsgemäßen Verfahren verwendet man im allgemeinen pro Mol Benzoesäure II 1 bis 3 Mol Bortrifluorid.

Das Molverhältnis Benzoesäure II: Aromat III beträgt in der Regel 1 : 0,5 bis 1 : 2.

Aufgrund der ausgezeichneten Löslichkeit der Bortrifluorid-Komplexe der Reaktanden kann die Menge an Nitrobenzol als Reaktionsmedium gering gehalten werden. Sie beträgt üblicherweise 100 bis 250 Gew.-%, bezogen auf die Summe des Gewichts von Benzoesäure II und Aromat III.

Das neue Verfahren zeichnet sich außerdem durch kurze Reaktionszeit und niedrige Reaktionstemperatur aus. Die Zielprodukte fallen im allgemeinen in hoher Reinheit an und können daher ohne weitere Reinigung weiterverwendet werden.

Die mittels des erfindungsgemäßen Verfahrens, das sowohl in kontinuierlicher als auch diskontinuierlicher Arbeitsweise durchgeführt werden kann, hergestellten Benzophenone sind als UV-Absorber oder als Monomere für hochtemperaturfeste Polymere geeignet.

Die folgenden Beispiele sollen die Erfindung näher erläutern.

### Beispiel 1

27,5 g Resorcin und 30,5 g Benzoesäure wurden in 100 ml Nitrobenzol suspendiert. In diese Suspension wurden dann 34 g Bortrifluorid eingegast. Das Reaktionsgemisch wurde auf 80°C erwärmt und 30 Minuten bei dieser Temperatur gehalten. Nach dem Abkühlen wurde mit Wasserdampf destilliert. Das Zielprodukt 2,4-Dihydroxybenzophenon kristallisierte beim Abkühlen aus der Mutterlauge aus. Ausbeute: 51,2 g (96 %); Reinheit gemäß HPLC: 99,8 %.

Die in der folgenden Tabelle aufgeführten Benzophenone wurden in analoger Weise erhalten.

**Tabelle**

| Beispiel Nr. | Benzoesäure II | Aromat III | Benzophenon I | Ausbeute [%] | Reinheit gem. HPLC [%] |
|---|---|---|---|---|---|
| 2 | 2,4-Dihydroxybenzoesäure | Resorcin | 2,2',4,4'-Tetrahydroxybenzophenon | 59 | 98,5 |
| 3. | 4-Hydroxybenzoesäure | Hydrochinon | 3',4,5'-Trihydroxybenzophenon | 64 | |

### Beispiel 4

220 g Resorcin und 308 g 2,4-Dihydroxybenzoesäure wurden in 1 l Nitrobenzol suspendiert und bei 25 bis 40°C 138 g Bortrifluorid eingeleitet (schwach exotherme Reaktion). Das Reaktionsgemisch wurde für 30 Minuten auf 80°C erwärmt und anschließend in einen auf 98°C vorgewärmten, mit 1,5 l Wasser beschichteten, kontinuierlich betriebenen Flüssig-Flüssig-Extraktor gegeben. Aus dem Extrakt wurde Wasser destillativ zurückgeführt und erneut zur Extraktion verwendet. Nach 10 Stunden wurde die wäßrige Phase abgetrennt, abgekühlt und das auskristallisierte Produkt abgesaugt. Ausbeute: 315 g (64 %); Reinheit gemäß HPLC: 99,6 %.

## Patentansprüche

1. Verfahren zur Herstellung von Benzophenonen der Formel I 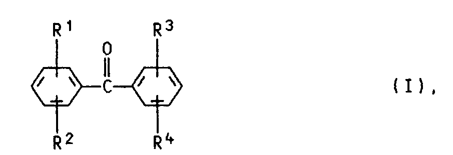 in der
R¹ und R² gleich oder verschieden sind und unabhängig voneinander jeweils Wasserstoff oder Hydroxy und
R³ und R⁴ jeweils Hydroxy bedeuten,
durch Umsetzung von Benzoesäuren der Formel II 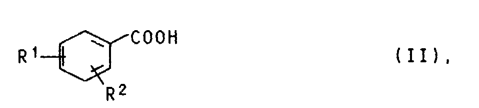 in der R¹ und R² jeweils die obengenannte Bedeutung besitzen, mit Aromaten aus der Benzolreihe, die der Formel III 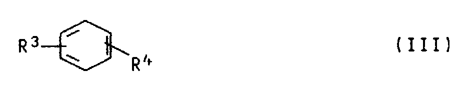 entsprechen, worin R³ und R⁴ jeweils die obengenannte Bedeutung besitzen, in Gegenwart von Bortrifluorid als Katalysator, dadurch gekennzeichnet, daß man die Umsetzung in Nitrobenzol als Reaktionsmedium durchführt.

2. Verfahren gemäß Anspruch 1, dadurch gekennzeichnet, daß man die Umsetzung bei einer Temperatur von 20 bis 200°C durchführt.

3. Verfahren gemäß Anspruch 1, dadurch gekennzeichnet, daß man pro Mol Benzoesäure der Formel II 1 bis 3 Mol Bortrifluorid verwendet.

## Claims

1. A process for preparing a benzophenone of the formula I 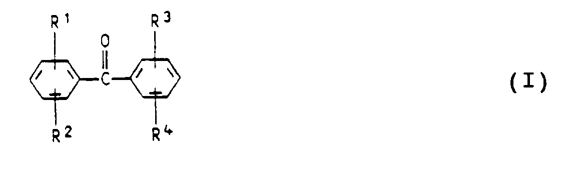 where
R¹ and R² are identical or different and each is independently of the other hydrogen or hydroxyl and
R³ and R⁴ are each hydroxyl,
by reacting a benzoic acid of the formula II 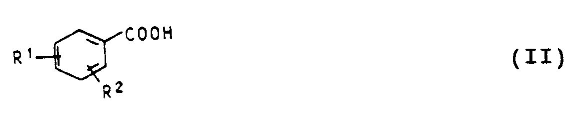 where R¹ and R² are each as defined above, with an aromatic from the benzene series which conforms to formula III 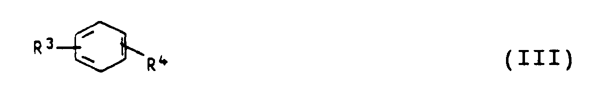 where R³ and R⁴ are each as defined above, in the presence of boron trifluoride as a catalyst in nitrobenzene as reaction medium.

2. A process as claimed in claim 1, wherein the reaction is carried out at from 20 to 200°C.

3. A process as claimed in claim 1, wherein from 1 to 3 moles of boron trifluoride are used per mole of benzoic acid of the formula II.

## Revendications

1. Procédé de préparation de benzophénones de formule I 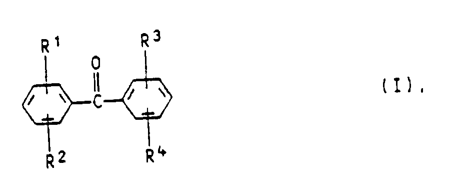 dans laquelle
R¹ et R² sont identiques ou différents et représentent chacun, indépendamment l'un de l'autre, un atome d'hydrogène ou un reste hydroxy et
R³ et R⁴ représentent chacun un reste hydroxy,
par réaction d'acides benzoïques de formule II 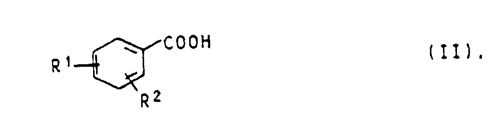 dans laquelle R¹ et R² ont chacun la signification susmentionnée, avec des composés aromatiques de la série du benzène qui répondent à la formule III 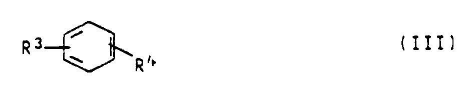 dans laquelle R³ et R⁴ ont chacun la signification susmentionnée, en présence de trifluorure de bore servant de catalyseur, caractérisé en ce qu'on conduit la réaction dans du nitrobenzène en tant que milieu réactionnel.

2. Procédé selon la revendication 1, caractérisé en ce qu'on conduit la réaction à une température de 20 à 200°C.

3. Procédé selon la revendication 1, caractérisé en ce qu'on utilise, par mole d'acide benzoïque de formule II, 1 à 3 moles de trifluorure de bore.
